# EUROPEAN PATENT APPLICATION

(11) **EP 4 282 408 A1**
(43) Date of publication of application: **29.11.2023**
(21) Application number: 22174877.5
(22) Date of filing: 23.05.2022
(51) Int. Cl.: A61K 31/19, A61K 31/4184, A61K 31/475, A61K 31/555, A61K 31/573, A61K 31/7068, A61K 39/00, A61P 35/00

(54) **METHOD FOR PRETREATING RELAPSING CANCER**

(71) Applicant: Valcuria AB, 223 81 Lund (SE)
(72) Inventor: SANDBERG, Mia, 234 38 LOMMA (SE); DROTT, Kristina, 222 29 LUND (SE)
(74) Representative: Brann AB

(57) **Abstract**

The present invention relates to a histone deacetylase (HDAC) inhibitor for use in the pretreatment of relapsing cancer prior to treatment of the relapsing cancer, wherein the HDAC inhibitor is selected from the group consisting of valproic acid, valproate semisodium, sodium valproate, magnesium valproate or mixtures thereof, and wherein the HDAC inhibitor is administered to a subject suffering from the relapsing cancer.

## Description

### FIELD OF INVENTION

The invention relates to the field of methods for pretreating relapsing cancer, i.e., where one or more compositions is administered to an individual in need of cancer treatment as a pretreatment prior to other treatments to enhance the effect, or mitigate the side-effects, of the treatments.

### BACKGROUND OF INVENTION

Cancer can be defined as an abnormal growth of cells which exhibit signs of uncontrolled proliferation and disturbed programmed cell death. From a classical view, sequential genetic events lead to malignant transformation, resulting in a cell clone that does not respect the integrity of other cells and tissues, and may eventually metastasize. Cancer can involve any tissue of the body and have many different forms in each body area.

Malignant lymphoma can be defined as a malignant transformation of the lymphatic cells of the hematopoietic system. Lymphomas can be divided into aggressive lymphomas and indolent lymphomas. Aggressive lymphomas are characterized by a rapid growth pattern and can have dramatic clinical features. However, aggressive lymphomas can reach a complete cure by treatment with chemotherapy, radiotherapy and monoclonal antibodies. In contrast, indolent lymphomas (e.g., follicular lymphomas) have a slow growth pattern, and usually a more modest clinical presentation. However, although indolent lymphomas cannot reach a complete cure by standard lymphoma treatment, they can sometimes be cured by allogeneic stem cell transplantation. The median survival time for follicular lymphomas is 8-10 years. Diffuse Large B Cell Lymphoma and Hodgkin lymphoma belong to the group of aggressive lymphomas, while follicular lymphoma and chronic lymphocytic leukaemia are indolent lymphomas. Myelomas consist of malignantly transformed plasmacells. They are related to indolent lymphomas but are usually considered an entity of their own. The prognosis is pessimistic, with a median survival time of 5-7 years.

One of the most frequent subtypes of malignant lymphoma is Diffuse Large B-cell Lymphoma (DLBCL) with an incidence of about 500 cases/year in Sweden. DLBCLs constitute 60-70% of the group of aggressive lymphomas. The median age at diagnosis is 70 years, and DLBCL is slightly more common in males than in females.

Standard first line treatment of DLBCL is chemotherapy consisting of a combination of cyclophosphamide, doxorubicin, vincristine and prednisone (CHOP). During recent years addition of the CD20 antibody rituximab has become international clinical standard (R-CHOP), leading to improved progression-free, event-free, disease-free and overall survival (Morrison, Expert Rev Anticancer Ther, 2008; 8(10): pp. 1651-1658). Still, since as many as 45 % of patients die from their disease, there is a pronounced clinical need to increase progression-free survival in DLBCL patients.

One important field in the study of cancer diseases is the regulation of DNA transcription. This is a complex process and the mechanisms involved are only partially known. Histone Deacetylases (HDACs) can regulate expression of tumour suppressor genes and activities of transcription factors involved in both cancer initiation and progression. HDACs act through alteration of either DNA or the structural components of chromatin by histone deacetylation, thus affecting the three-dimensional conformation of DNA without changing or interrupting its sequence (epigenetic modifications). It has also been suggested that they may alter the sensitivity to DNA damaging chemotherapy through modulation of chromatin structure. Along these lines, several in vitro studies have suggested that inhibition of HDACs can synergize with chemotherapy.

Therefore, numerous HDAC inhibitors have been developed during the recent years. They can be divided into four classes: hydroxamic acids/carbamic acids, cyclic peptides, aliphatic acids and benzamides. Examples of HDAC inhibitors which have been approved for treatment of cancer include vorinostat and romidepsin which are approved for the treatment of cutaneous T-cell lymphoma lymphoma by the FDA (Food and drug administration), and which are currently evaluated in the treatment of other malignancies.

The clinically most well-known HDAC inhibitor is the anticonvulsant valproic acid, which has been utilized in the treatment of epilepsy since the 1970s. Valproic acid belongs to the aliphatic acid class of inhibitors.

The present inventors have previously, see WO2012/128709, shown that HDAC inhibitors in combination with steroids are useful when being administered to a human in need of cancer treatment as a pretreatment prior to other treatments, the result being that the effect of the treatment is enhanced.

Despite the promise of more effective treatment of cancer made by the advent of pretreatment using a HDAC inhibitor as taught by WO2012/128709, there are still difficulties to overcome to more fully realize the potential of cancer pretreatment using an HDAC inhibitor.

In particular, there remains a need for effective strategies to treat relapsing disease, i.e., where patients have undergone a first type of treatment, but the cancer remains or reappears. Typically, the first type of treatment may not be suitable for treating the relapsing cancer, and new treatments are needed for this patient group.

### SUMMARY OF THE INVENTION

The invention relates to the finding that valproic acid and its derivatives can be administered as a pretreatment, prior to a treatment, of relapsing cancer.

Thus, in a first aspect the present invention relates to a histone deacetylase (HDAC) inhibitor for use in the pretreatment of relapsing cancer prior to treatment of the relapsing cancer, wherein the HDAC inhibitor is selected from the group consisting of valproic acid, valproate semisodium, sodium valproate, magnesium valproate or mixtures thereof, and wherein the HDAC inhibitor is administered to a subject suffering from the relapsing cancer.

A corresponding second aspect the present invention relates to a method of pretreating relapsing cancer, wherein a histone deacetylase HDAC inhibitor selected from the group consisting of valproic acid, valproate semisodium, sodium valproate, magnesium valproate or mixtures thereof, is administered to a subject suffering from the relapsing cancer.

A corresponding third aspect of the present invention relates to the use of a histone deacetylase HDAC inhibitor selected from the group consisting of valproic acid, valproate semisodium, sodium valproate, magnesium valproate or mixtures thereof in the manufacturing of a medicament for pretreating relapsing cancer

A fourth aspect of the present invention relates to a histone deacetylase (HDAC) inhibitor and a therapeutic agent for use in the combination treatment of relapsing cancer, wherein the HDAC inhibitor is selected from the group consisting of valproic acid, valproate semisodium, sodium valproate, magnesium valproate or mixtures thereof, wherein the therapeutic agent is selected from the list consisting of bendamustine, gemcitabine and carboplatin, and wherein the HDAC inhibitor is administered to a subject suffering from the relapsing cancer prior to the therapeutic agent being administered to the subject suffering from the relapsing cancer.

A corresponding fifth aspect of the present invention relates to a method of combination treatment of relapsing cancer, the method comprising the steps of:
- pretreating the relapsing cancer by administrating a histone deacetylase HDAC inhibitor selected from the group consisting of valproic acid, valproate semisodium, sodium valproate, magnesium valproate or mixtures thereof to a subject suffering from the relapsing cancer, and
- treating the relapsing cancer by administrating a therapeutic agent selected from the list consisting of bendamustine, gemcitabine and carboplatin to the subject suffering from the relapsing cancer.

A sixth aspect of the present invention relates to a kit for pretreating and treating relapsing cancer comprising:
- one or more doses of an HDAC inhibitor selected from the group consisting of valproic acid, valproate semisodium, sodium valproate, magnesium valproate or mixtures thereof,
- one or more doses of a therapeutic agent selected from the list consisting of bendamustine, gemcitabine and carboplatin.

### BRIEF DESCRIPTION OF THE DRAWINGS

Fig. 1A-1B show cell proliferation results for a WSU-NHL cell line after 48-hour pretreatment using valproic acid followed by 24-hour treatment with gemcitabine, bendamustine and carboplatin.
Fig. 2A-2B show cell proliferation results for a WSU-NHL cell line after 48-hour pretreatment using valproic acid followed by 48-hour treatment with gemcitabine, bendamustine and carboplatin.
Fig. 3A-3B show cell proliferation results for a SU-DHL cell line after 48-hour pretreatment using Valproic acid followed by 24-hour treatment with gemcitabine, bendamustine and carboplatin.

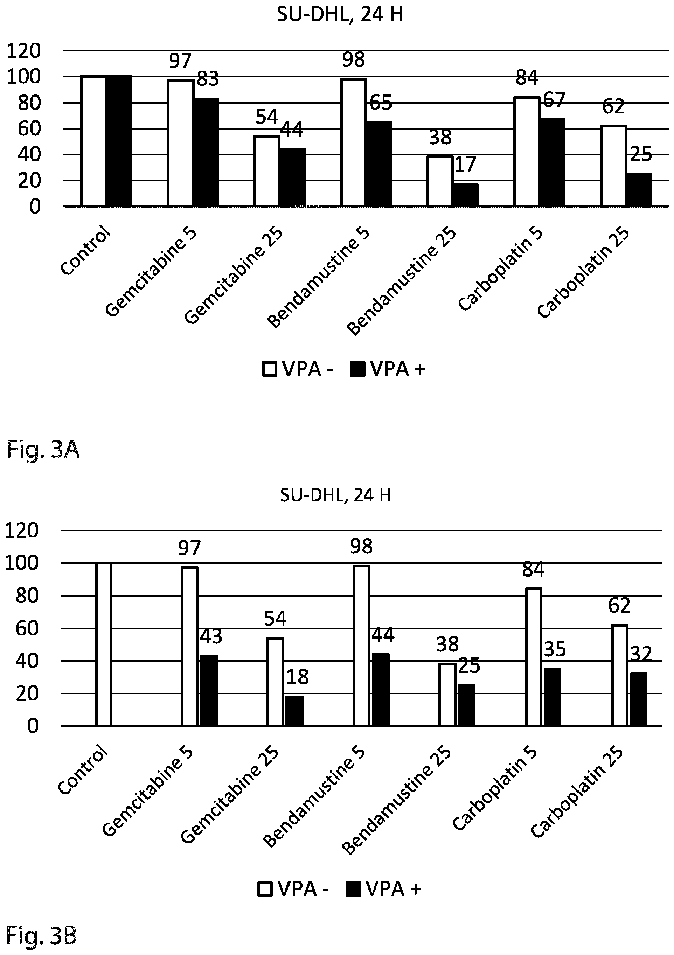

Fig. 3A-3B show cell proliferation results for a SU-DHL cell line after 48-hour pretreatment using valproic acid followed by 48-hour treatment with gemcitabine, bendamustine and carboplatin.

### DETAILED DESCRIPTION OF THE INVENTION

A first aspect the present invention relates to a histone deacetylase (HDAC) inhibitor for use in the pretreatment of relapsing cancer prior to treatment of the relapsing cancer, wherein the HDAC inhibitor is selected from the group consisting of valproic acid, valproate semisodium, sodium valproate, magnesium valproate or mixtures thereof, and wherein the HDAC inhibitor is administered to a subject suffering from the relapsing cancer.

A corresponding second aspect the present invention relates to a method of pretreating relapsing cancer, wherein a histone deacetylase HDAC inhibitor selected from the group consisting of valproic acid, valproate semisodium, sodium valproate, magnesium valproate or mixtures thereof, is administered to a subject suffering from the relapsing cancer.

A corresponding third aspect of the present invention relates to the use of a histone deacetylase HDAC inhibitor selected from the group consisting of valproic acid, valproate semisodium, sodium valproate, magnesium valproate or mixtures thereof in the manufacturing of a medicament for pretreating relapsing cancer.

The administration of the HDAC inhibitor is preferably systemic, such as by oral administration or parenteral (intravenous, subcutaneous, and intramuscular) administration. The HDAC inhibitor may be formulated in a pharmaceutical composition. The pharmaceutical composition may be in the form selected from the group consisting of granulates, powders, tablets, coated tablets, microcapsules, microgranulates and effervescent forms. The pharmaceutical composition is preferably formulated for systemic administration. Consequently, the preferred administration routes are oral administration and parenteral (intravenous, subcutaneous, and intramuscular) administration. The pharmaceutical composition may comprise one or more other pharmaceutical acceptable pharmaceutical ingredient, such as a pharmaceutically acceptable diluent, carrier, excipient or buffer. "Pharmaceutically acceptable" means a non-toxic compound that does not decrease the effectiveness of the biological activity of the active ingredients.

The term "buffer" is intended to mean an aqueous solution containing an acid-base mixture with the purpose of stabilising pH. Examples of buffering agents are magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

The term "diluent" is intended to mean an aqueous or non-aqueous solution with the purpose of diluting the compounds in the pharmaceutical preparation. The diluent may be one or more of saline, water, polyethylene glycol, propylene glycol or ethanol.

The excipient may be one or more of carbohydrates, surfactants, polymers, lipids and minerals. Examples of carbohydrates include lactose, sucrose, mannitol, and cyclodextrines, which are added to the composition, e.g., for facilitating lyophilisation. Examples of polymers are starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone, all of different molecular weight, which are added to the composition, e.g., for viscosity control, for achieving bioadhesion, or for protecting the lipid from chemical and proteolytic degradation. Examples of lipids are fatty acids, phospholipids, mono-, di-, and triglycerides, ceramides, sphingolipids and glycolipids, all of different acyl chain length and saturation, egg lecithin, soy lecithin, hydrogenated egg and soy lecithin, which are added to the composition for reasons similar to those for polymers. Examples of minerals are talc, magnesium oxide, zinc oxide and titanium oxide, which are added to the composition to obtain benefits such as reduction of liquid accumulation or advantageous pigment properties.

Examples of suitable aqueous and nonaqueous carriers which may be employed in the pharmaceutical composition include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions.

If the product is a tablet it may comprise at least one additive selected from the group comprising binders, lubricants, emulsifiers fillers, surfactants (e.g., polysorbate 80 and sodium lauryl sulfate), flavours, aromas (examples of ingredients giving taste) (such as orange, lemon, bergamon, grapefruit, banana, apricot and strawberry) and colours, including natural or synthetic ones, vitamins, sweeteners (examples of ingredients giving taste) (acesulfame potassium, sodium saccharin, aspartame, stevia and sucralose), nutritional additives (e.g, antioxidants, peptides), and mixtures thereof.

Additionally, the tablet may contain various lubricants suitable for use in the composition including water dispersible, water soluble, water insoluble lubricants and combinations thereof. Examples of useful water-soluble lubricants include sodium benzoate, polyethylene glycol, L-leucine, adipic acid, and combinations thereof.

The tablet may also include water insoluble lubricants including, e.g., stearates (e.g., magnesium stearate, calcium stearate and zinc stearate), oils (e.g., mineral oil, hydrogenated and partially hydrogenated vegetable oils, and cotton seed oil) and combinations thereof.

In the context of the present invention pretreatment is to be understood as a treatment performed prior to other treatments to enhance the effect, or mitigate the side-effects, of the other treatments. More particularly pretreatment of relapsing cancer encompasses sensitizing cancer cells prior to these cells being treated with other treatments thereby enhancing the effect of the other treatments. The pretreatment itself typically has only a limited effect on the cancer cells. Rather it is the combination of the pretreatment and the other treatments that provide an effective treatment of the relapsing cancer cells.

The relapsing cancer is preferably selected from the group consisting of sarcoma, malignant melanoma, skin cancer, estrogen receptor-dependent and independent breast cancer, ovarian cancer, prostate cancer, renal cancer, colon and colorectal cancer, pancreatic cancer, head and neck cancer, small cell and non-small cell lung carcinoma, and cancer of blood cells.

The treatment of the relapsing cancer should be selected based on the type of cancer that the subject is suffering from. Treatments include inter alia chemotherapy and/or immunotherapy. Chemotherapy may comprise administration of CHOP, a combination of cyclophosphamide, doxorubicin, vincristine and prednisone, which may be administered in amounts of 750 +/- 10% mg/m² of cyclophosphamide, 50 +/- 10% mg/m² of doxorubicin, 1.4 +/- 10% mg/m² of vincristine and 50 +/- 10% mg/m² of prednisone, wherein m² refers to the body surface of the human.

Immunotherapy may comprise administering an antibody, monoclonal antibody or a functional fragment thereof, such as rituximab, ofatumumab, GA101, tositumumab, ibritumumab, ocraluzumab, veltuzumab, epratuzumab, FTBA05, AME-133V or R603. All the above-mentioned antibodies bind to CD20 present on B-cells. The antibodies may be administrated in an amount of 375 +/- 10% mg/m².

The treatment is typically performed using a therapeutic agent. The therapeutic agent may comprise one or more chemotherapeutic and/or Immunotherapeutic substances as described above. Other examples of treatment and therapeutic agents are surgery, radiation, and gene therapy.

The HDAC inhibitor is administered to the subject. The subject is preferably a mammal, more preferably a human.

The HDAC inhibitor should be administered in a pharmaceutically effective dose. By "pharmaceutically effective dose" is meant a dose that is sufficient to produce the desired effects in relation to the condition for which it is administered. The exact dose is dependent on the manner of administration, the nature and severity of the disorder, and on the general health, sex, age and body weight of the patient. The total amount of the HDAC inhibitor should be selected so as to provide a plasma concentration of the HDAC inhibitor in the patient at or above the desired concentration for the specific HDAC inhibitor.

The desired plasma concentration for valproic acid is expected to be in the range of 500 to 2500 µM, preferably 500 to 1500 µm, such as 600 to 1000 µm, during the pretreatment.

The HDAC inhibitor may be administered once daily, such as during morning time, about 5 to 8 in the morning. However, the administration of the HDAC inhibitor may occur 1, 2, 3, 4 or even up to 5 times daily. The administration of the HDAC inhibitor may be at least 24- 72 hours prior to the treatment, such as 30-60, 40-50 or 48 hours prior to the treatment.

Accordingly, valproic acid may be administrated orally or intravenously in ranges from about 500 mg to about 15000 mg per day, such as from about 4000 mg to about 15000 mg per day, such as from about 400 mg to about 3000 mg per day. For example, oral dosages can be about 800, about 1600, about 2400, about 3000, about 6000, about 9000, about 15000 mg per day. The amount may be administered in one or more doses. Preferably the HDAC inhibitor is dosed based on the body weight of the human (so as to obtain the desired plasma concentration) but can include also the body surface area of the human. The body surface area of the human is preferably calculated using the following formula (Dubois & Dubois): 0.20247 x height (m)^{0.725} x weight (kg)^{0.425}.

Preferably the relapsing cancer is selected from the group consisting of relapsing diffuse large B cell lymphoma (DLBCL), relapsing follicular lymphoma, relapsing chronic lymphocytic leukaemia, and relapsing Hodgkin lymphoma. More preferably the relapsing cancer is diffuse large B cell lymphoma (DLBCL).

Preferably, the treatment of the relapsing cancer comprises administration of one or more therapeutic agents selected from the list consisting of bendamustine, gemcitabine and carboplatin.

Bendamustine (1*H*-Benzimidazole-2-butanoic acid, 5-[bis(2-chloroethyl)amino]-1-methyl-, CAS No. 16506-27-7) is sold under inter alia the brand name Treanda. It is a chemotherapy medication used in the treatment of chronic lymphocytic leukemia (CLL), multiple myeloma, and non-Hodgkin's lymphoma. It is given by injection.

Gemcitabine (Cytidine, 2'-deoxy-2',2'-difluoro-, CAS No. 95058-81-4)is a chemotherapy medication. It treats cancers including testicular cancer, breast cancer, ovarian cancer, non-small cell lung cancer, pancreatic cancer, and bladder cancer. It is administered by intravenous infusion.

Carboplatin (Platinum, diammine[1,1-cyclobutanedicarboxylato(2-)-KO,KO"]-, (*SP-*4-2)-, CAS No. 41575-94-4) is a chemotherapy medication used to treat inter alia ovarian cancer, lung cancer, head and neck cancer, brain cancer, and neuroblastoma. It is used by injection.

Typically, the relapsing cancer is a recurrence of a primary or previous cancer. A recurrence means that the cancer has come back after a period during which the cancer could not be detected. The relapsing cancer may be positioned in the same place as the primary cancer (tumor) or may appear at a different position in the body of the subject. Primary cancer and previous cancer refer to a previous, typically a first, occurrence of cancer in the subject.

Preferably the primary or previous cancer is selected from the group consisting of diffuse large B cell lymphoma (DLBCL), follicular lymphoma, chronic lymphocytic leukaemia, and Hodgkin lymphoma. More preferably the primary or previous cancer is diffuse large B cell lymphoma (DLBCL).

Preferably the primary or previous cancer was treated with chemotherapy and/or immunotherapy. Chemotherapy comprises the administration of one or more chemotherapeutic agents. Immunotherapy is treatment that uses certain parts of the subject's immune system to fight cancer. Immunotherapy may comprise administration of immunotherapeutic agents such as checkpoint inhibitors, cytokines, immunomodulators, cancer vaccines, monoclonal antibodies, and oncolytic viruses, as well as procedures such as chimeric antigen receptor (CAR) T-cell therapy.

Preferably the chemotherapy comprises the combination of cyclophosphamide, doxorubicin, vincristine and prednisone (CHOP), Preferably the immunotherapy comprises a CD20 antibody, preferably rituximab.

Preferably the relapsing cancer is relapsing diffuse large B cell lymphoma (DLBCL), wherein the primary or previous cancer is diffuse large B cell lymphoma (DLBCL), and wherein the subject suffering from the relapsing cancer has been treated for the primary or previous cancer using the combination of cyclophosphamide, doxorubicin, vincristine and prednisone (CHOP), or the combination of a CD20 antibody, preferably rituximab, cyclophosphamide, doxorubicin, vincristine and prednisone (R-CHOP).

Preferably a steroid is also administered to the subject suffering from the relapsing cancer as part of the pretreatment of the relapsing cancer.

The steroid may be administered before, after, or at the same time as the HDAC inhibitor. The steroid may be administered in its own pharmaceutical composition or may be comprised in the pharmaceutical composition holding the HDAC inhibitor. The steroid may increase the effect of the pretreatment

Preferably the steroid is selected from the group consisting of prednisone, prednisolone, dexamethasone or betamethasone, and wherein the steroid preferably is selected from the group consisting of prednisone and prednisolone.

Prednisone or prednisolone may be administered in an amount of 20 to 200 mg per day, such as 50-200, 100-150, 30, 40, 50, 60, 70, 80, 90, 100, 110, 120, 130, 140, 150, 160, 170,180, 190 or 200 mg per day. The amount may be administered in one or more doses.

Betamethasone may be administered in an amount of 4 to 32 mg per day, such as 10-25, 10-20, 5, 6, 7, 8, 9, 10, 11, 12, 13, 14, 15, 16, 17, 18, 19, 20, 21, 22, 23, 24, 25, 26, 27, 28, 29, 30, 31 or 32 mg per day. The amount may be administered in one or more doses.

Dexamethasone may be administered in an amount of 10 to 80 mg per day, such as 20-70, 10, 20, 30, 40, 50, 60, 70 or 80 mg. The amount may be administered in one or more doses.

A fourth aspect of the present invention relates to a histone deacetylase (HDAC) inhibitor and a therapeutic agent for use in the combination treatment of relapsing cancer, wherein the HDAC inhibitor is selected from the group consisting of valproic acid, valproate semisodium, sodium valproate, magnesium valproate or mixtures thereof, wherein the therapeutic agent is selected from the list consisting of bendamustine, gemcitabine and carboplatin, and wherein the HDAC inhibitor is administered to a subject suffering from the relapsing cancer prior to the therapeutic agent being administered to the subject suffering from the relapsing cancer.

A corresponding fifth aspect of the present invention relates to a method of combination treatment of relapsing cancer, the method comprising the steps of:
- pretreating the relapsing cancer by administrating a histone deacetylase HDAC inhibitor selected from the group consisting of valproic acid, valproate semisodium, sodium valproate, magnesium valproate or mixtures thereof to a subject suffering from the relapsing cancer, and
- treating the relapsing cancer by administrating a therapeutic agent selected from the list consisting of bendamustine, gemcitabine and carboplatin to the subject suffering from the relapsing cancer.

A sixth aspect of the present invention relates to a kit for pretreating and treating relapsing cancer comprising:
- one or more doses of an HDAC inhibitor selected from the group consisting of valproic acid, valproate semisodium, sodium valproate, magnesium valproate or mixtures thereof,
- one or more doses of a therapeutic agent selected from the list consisting of bendamustine, gemcitabine and carboplatin.

The doses are preferably provided in a suitable container and/or with suitable packaging, and the kit preferably further comprises instructions for how and when to administer the doses. Thus, the kit may for example comprise one or more blisters for holding the doses. Preferably the kit comprises a blister pack comprising a plurality of collapsible blisters sealed by a frangible sealing sheet, each of the blisters holding one or more doses. The kit may further include additional parts, including, for example, appropriate solutions for dilution (e.g., physiological saline solution, glucose solution, etc.), reagents (e.g., for adjusting pH), and devices (e.g., bags, tubes, syringes, needles, transfer sets) for assembly and use.

Preferably the kit further comprises:
- one or more doses of a steroid selected from the group consisting of prednisone, prednisolone, dexamethasone or betamethasone, wherein the steroid preferably is selected from the group consisting of prednisone and prednisolone.

### EXAMPLES

### Example 1: Valproic acid potentiates the effect of bendamustine, gemcitabine and carboplatin on proliferation of B cell lymphoma

In this example the combined effect of valproic acid and three different compounds as well as the single treatment on the growth of two cell lines were measured by WST 1 assay.

The cell lines used where:
SU DHL 8: human B cell lymphoma, and
WSU NHL: human B cell lymphoma

The WST 1 colorimetric assay comprised providing suspended cells (5000 cells/well) in the wells of a well plate. Tetrazolium salt (WST 1) was added to the cells and thereby colored the fluid in the wells red. Live cells reduce the tetrazolium salt via intermediate electron acceptors and forms a formazan product and the fluid in the cells turn yellow. The extent of color change reflects the proliferation of the cells and is read out as optical absorbance at 420 nm. Both cell lines were determined to be able to tolerate concentrations of up to 1% Dimethyl sulfoxide (DMSO), and this DMSO concentration was used as negative control. Additionally, 10% DMSO was used as positive control.

The cells of each cell line were incubated in a pretreatment step with 1 mM valproic acid for 48 hours followed by a treatment step with the respective compound for 24 or 48 hours, as shown in table 1 below:

**Table 1**

| Treatment step | Compound | Time | Concentration |
|---|---|---|---|
| Pretreatment | Valproic acid | 48 h | 1 mM |
| Treatment | Bendamustine | 24 h / 48 h | 5 µM / 25 µM |
| Treatment | Gemcitabine | 24 h / 48 h | 5 nM / 25 nM |
| Treatment | Carboplatin | 24 h / 48 h | 5 µM / 25 µM |

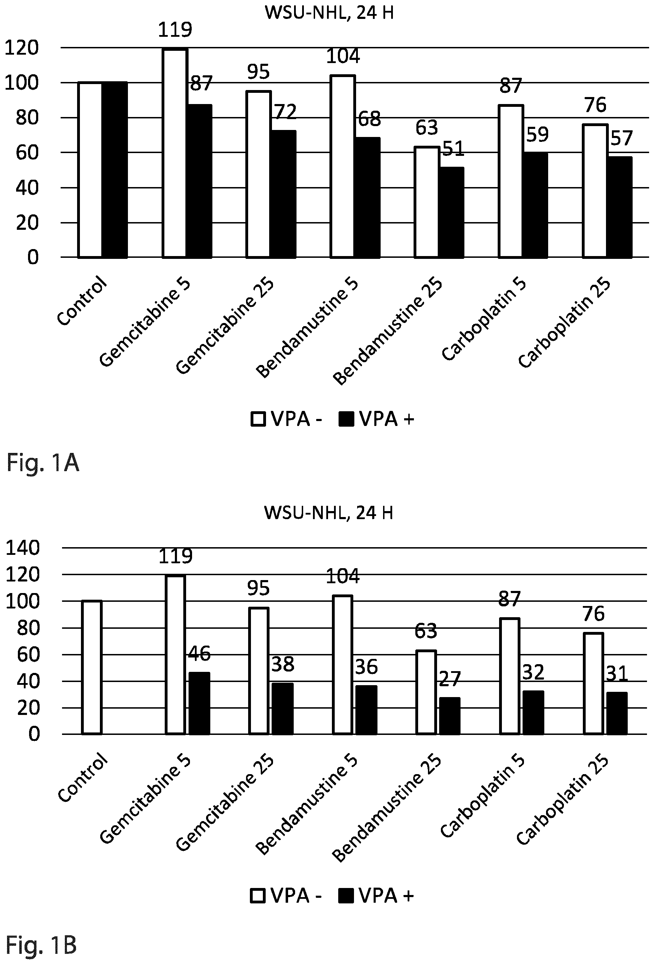

Fig. 1A shows the result on cell proliferation of pretreatment of cells from the WSU-NHL cell line followed by 24 h treatment using the respective compounds. The Y-axis represents precent growth of treated cells normalized to values obtained with 1% DMSO treated cells in the absence or presence of valproic acid (VPA). As seen in the graph, 48h pretreatment with valproic acid increased the effect of the 24h treatment using the respective compounds.

Fig. 1B shows the result on cell proliferation of pretreatment of cells from the WSU-NHL cell line followed by 24 h treatment using the respective compounds. Here, the Y axis represents percent growth of treated cells normalized to values obtained with untreated control cells. As seen in the graph, 48h pretreatment with valproic acid increased the effect of the 24h treatment using the respective compounds. Especially significant effects are seen for the 5 nM level of gemcitabine and 5 µM of bendamustine.

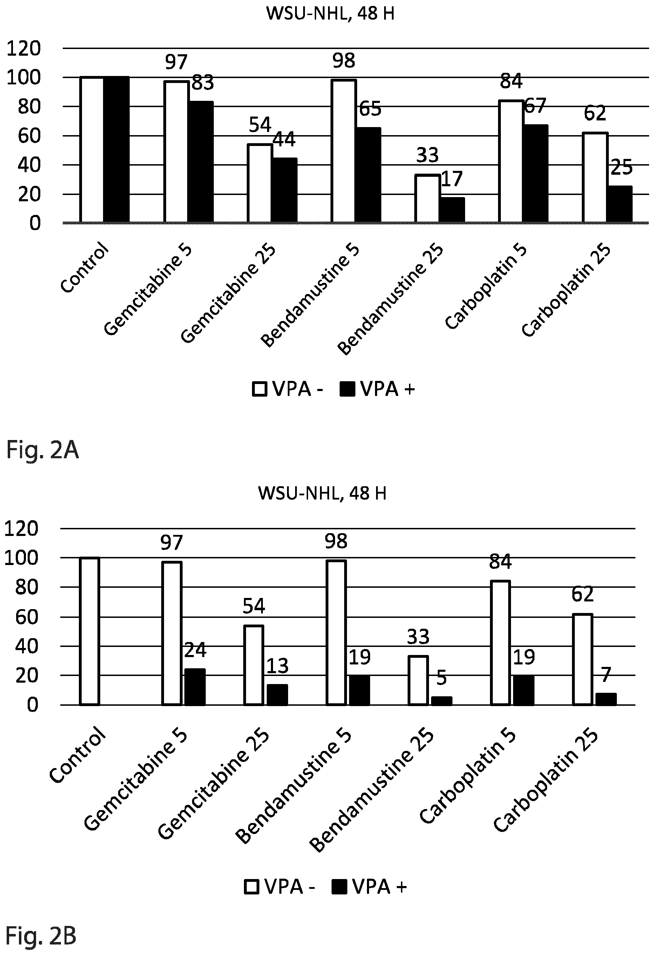

Fig. 2A shows the result on cell proliferation of pretreatment of cells from the WSU-NHL cell line followed by 48h treatment using the respective compounds. The Y-axis represents precent growth of treated cells normalized to values obtained with 1/ DMSO treated cells in the absence or presence of valproic acid (VPA). As seen in the graph, 48h pretreatment with valproic acid increased the effect of the 48h treatment using the respective compounds.

Fig. 2B shows the result on cell proliferation of pretreatment of cells from the WSU-NHL cell line followed by 48h treatment using the respective compounds. Here, the Y axis represents percent growth of treated cells normalized to values obtained with untreated control cells. As seen in the graph, 48h pretreatment with Valproic acid increased the effect of the 48h treatment using the respective compounds. Especially significant effects are seen for the 5 nM level of gemcitabine, and 5µM of bendamustine and carboplatin.

Fig. 3A shows the result on cell proliferation of pretreatment of cells from the SU-DHL cell line followed by 24 h treatment using the respective compounds. The Y-axis represents precent growth of treated cells normalized to values obtained with 1% DMSO treated cells in the absence or presence of valproic acid (VPA). As seen in the graph, 48h pretreatment with valproic acid increased the effect of the 24h treatment using the respective compounds.

Fig. 3B shows the result on cell proliferation of pretreatment of cells from the SU-DHL cell line followed by 24 h treatment using the respective compounds. Here, the Y-axis represents percent growth of treated cells normalized to values obtained with untreated control cells. As seen in the graph, 48h pretreatment with valproic acid increased the effect of the 24h treatment using the respective compounds.

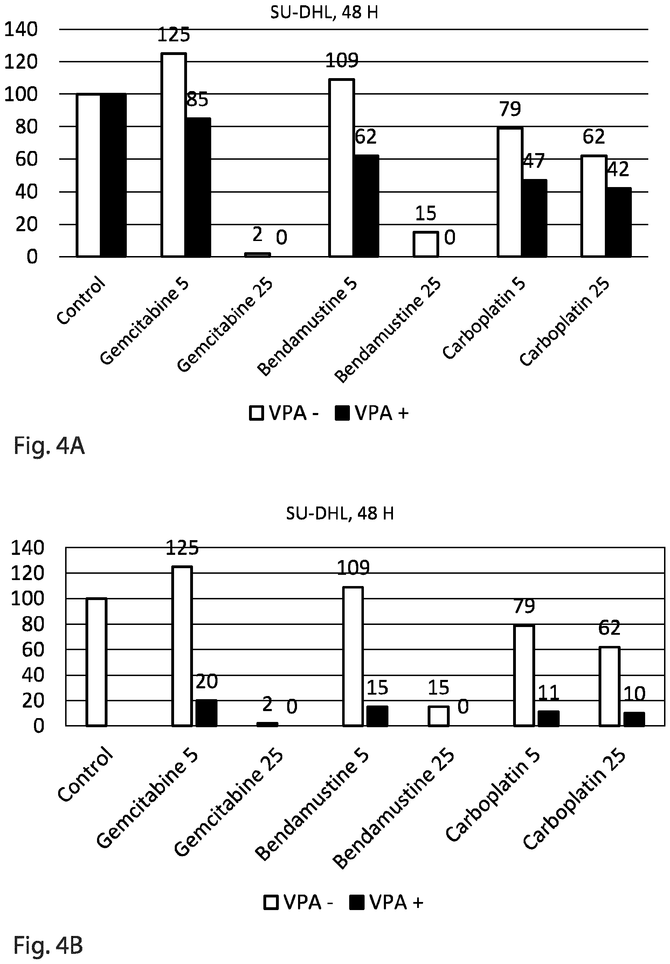

Fig. 4A shows the result on cell proliferation of pretreatment of cells from the SU-DHL cell line followed by 48h treatment using the respective compounds. The Y-axis represents precent growth of treated cells normalized to values obtained with 1/ DMSO treated cells in the absence or presence of valproic acid (VPA). As seen in the graph, 48h pretreatment with valproic acid increased the effect of the 48h treatment using the respective compounds. The combination of VPA and 25 nM of gemcitabine and 25 µM bendamustine killed all cancer cells.

Fig. 4B shows the result on cell proliferation of pretreatment of cells from the SU-DHL cell line followed by 48h treatment using the respective compounds. Here, the Y-axis represents percent growth of treated cells normalized to values obtained with untreated control cells. As seen in the graph, 48h pretreatment with valproic acid increased the effect of the 48h treatment using the respective compounds. The combination of VPA and carboplatin (at both 5 and 25 µM) leave about 10 cells alive.

As seen from the above examples and the figures, valprocic acid (VPA) is effective as pretreatment for later treatment using the therapeutic agents gemcitabine, bendamustine and carboplatin, which agents are used for treating relapsing cancer.

## Claims

1. A histone deacetylase (HDAC) inhibitor for use in the pretreatment of relapsing cancer prior to treatment of the relapsing cancer, wherein the HDAC inhibitor is selected from the group consisting of valproic acid, valproate semisodium, sodium valproate, magnesium valproate or mixtures thereof, and wherein the HDAC inhibitor is administered to a subject suffering from the relapsing cancer.

2. The histone deacetylase (HDAC) inhibitor for use according to claim 1, wherein the relapsing cancer is selected from the group consisting of relapsing diffuse large B cell lymphoma (DLBCL), relapsing follicular lymphoma, relapsing chronic lymphocytic leukaemia, and relapsing Hodgkin lymphoma.

3. The histone deacetylase (HDAC) inhibitor for use according to any preceding claim, wherein the treatment of the relapsing cancer comprises administration of one or more therapeutic agents selected from the list consisting of bendamustine, gemcitabine and carboplatin.

4. The histone deacetylase (HDAC) inhibitor for use according to any preceding claim, wherein the relapsing cancer is a recurrence of a primary or previous cancer.

5. The histone deacetylase (HDAC) inhibitor for use according to claim 4, wherein the primary or previous cancer is selected from the group consisting of diffuse large B cell lymphoma (DLBCL), follicular lymphoma, chronic lymphocytic leukaemia, and Hodgkin lymphoma.

6. The histone deacetylase (HDAC) inhibitor for use according to any preceding claim, wherein the primary or previous cancer was treated with chemotherapy and/or immunotherapy.

7. The histone deacetylase (HDAC) inhibitor for use according to any preceding claim, wherein the relapsing cancer is relapsing diffuse large B cell lymphoma (DLBCL), wherein the primary or previous cancer is diffuse large B cell lymphoma (DLBCL), and wherein the subject suffering from the relapsing cancer has been treated for the primary or previous cancer using the combination of cyclophosphamide, doxorubicin, vincristine and prednisone (CHOP), or the combination of a CD20 antibody, preferably rituximab, cyclophosphamide, doxorubicin, vincristine and prednisone (R-CHOP).

8. The histone deacetylase (HDAC) inhibitor for use according to any preceding claim, wherein a steroid is also administered to the subject suffering from the relapsing cancer as part of the pretreatment of the relapsing cancer.

9. The histone deacetylase (HDAC) inhibitor for use according to claim 8, wherein the steroid is selected from the group consisting of prednisone, prednisolone, dexamethasone or betamethasone, and wherein the steroid preferably is selected from the group consisting of prednisone and prednisolone.

10. A kit for pretreating and treating relapsing cancer comprising:
- one or more doses of an HDAC inhibitor selected from the group consisting of valproic acid, valproate semisodium, sodium valproate, magnesium valproate or mixtures thereof,
- one or more doses of a therapeutic agent selected from the list consisting of bendamustine, gemcitabine and carboplatin.

11. The kit according to claim 11, further comprising:
- one or more doses of a steroid selected from the group consisting of prednisone, prednisolone, dexamethasone or betamethasone, wherein the steroid preferably is selected from the group consisting of prednisone and prednisolone.
